# EUROPEAN PATENT APPLICATION

(11) **EP 1 982 999 A1**
(43) Date of publication of application: **22.10.2008**
(21) Application number: 07106250.9
(22) Date of filing: 16.04.2007
(51) Int. Cl.: C07K 16/04, C07K 16/16, A61K 35/20, A61K 39/395, A23C 9/20, A61K 47/48

(54) **Milk derived antigen specific antibodies, methods of preparation and uses thereof**

(71) Applicant: Friesland Brands B.V., 7943 PE Meppel (NL)
(72) Inventor: van Neerven, Ruprecht Jules Joost, 1321 HC Almere (NL)
(74) Representative: van Loon, C.J.J.

(57) **Abstract**

The invention relates to means and methods for obtaining an antigen specific antibody from milk from non-human mammals that have not been immunized with said antigen prior to collecting said milk. Non-human mammal derived antigen specific antibody was found to cross the mucosal side of the intestinal tract of an individual. The invention provides novel means and methods for the treatment of disease through antigen specific non-human mammal antibodies. Further provided are preparations for oral administration comprising said antigen specific antibody of a ruminant.

## Description

The invention relates to the field of antibodies. In particular the invention relates to milk-derived antigen specific antibodies, methods of preparation and uses thereof. Such uses entail for instance, in infant nutrition, in diagnostic and medical uses.

There are several ways to enhance human immunity by providing dairy foods. Mother's milk and cow's milk contain antibodies that are specific for a wide range of potential pathogenic microorganisms, viruses and other antigens. These antibodies protect the infant that is not yet able to mount an effective immune response against infectious agents that the mother has already been exposed to. IgA is the predominant antibody in human milk, but IgG₁ is the predominant antibody isotype found in bovine milk. The IgA in human milk is generally known to exert its effect in the intestine, whilst the mother transfers IgG to the baby via the placenta before birth. Most ruminants, pigs and rodents actively transport -a portion of the IgG present in milk over the intestinal epithelium into the blood. These animals thus, like humans, also have antibodies in the milk of lactating females, particularly in the first milk after birth (colostrum) albeit that the actual function of these antibodies in the neonate has additional properties as compared to antibodies in the milk of humans.

As ruminants produce milk that contains antibodies, the milk or antibody preparations thereof are considered for use in humans to supplement or replace the function of mother milk. To enable collection of antigen specific antibodies in the milk ruminant are typically immunized with the particular antigen for which antibodies are desired. The antigen is typically a human pathogen, for instance an intestinal microbe or virus and the antibodies in the milk of these immunized animals are collected. The collected antibodies are intended to bind to and neutralize or otherwise impair the human pathogen in the intestine of individuals ingesting the antibodies.

Apart from milk, colostrum is another source for isolating bovine antibodies. Bovine colostrum is the first milk produced by a cow just before and shortly after giving birth to a calf. Colostrum contains very high levels of bovine antibodies, up to 50-100 mg/ml (vs around 1 mg/ml for normal milk). IgG₁ is the predominant antibody isotype in bovine colostrum and in milk. As mentioned above, the immunoglobulins in colostrum are absorbed into the blood by calfs to protect them against infection. The antibody levels in colostrums are about 50-100 fold higher than in normal milk. For this reason colostrum of hyperimmunized cows has been used as a source of bovine antibodies to treat and prevent diarrhea in a number of studies showing significant effects on diarrhea symptoms. (Clin infect dis 1998 26:1324-9 adults; Acta pediatr. 1995 84:996-1001 infants; Acta paediatr. 2001 90 1373-8 children 1-12 y; J.Pediatr.Gastroenterol Nutr 1999 29:452-6). Some of these studies are reviewed in Indian J. Pediatr. 2005 72:849-52.
In addition to the use in preventing/treating diarrhea, colostrum is also used as a supplement by athletes (eur.J.Nutrition 2003 42:228-232 and Int.J.Sport Ntr.Excerc Metab 2006 16:47-64. J. Appl Physiol 2006 93:732-39).
In another study (JT van Dissel at al J.Med.Microbiol 2005 54-197-205) a 40% whey protein concentrate of cows immunized with Clostridium difficile was used to prevent relapses of C. difficile diarrhea - Results are preliminary, but no adverse events noted due to MucoMilk (Scand J.Gastroenterol 2000 35:711-8; N.Engl J Med 1988 318:1240-3.

These data show that colostrum from cows contain high antibody levels and that oral administration of these antibodies in adults and infants is without adverse effect, in at least these studies, indicating that the use of cow antibodies in food is safe.

Example 4 and example 5 of EP015227081 indicate that no serum sickness arises after ingestion of bovine milk and injection of purified IgG in humans. Rabbits drinking milk before the injection with IgG do not produce anti bovine IgG antibodies. These examples again indicate safety and oral tolerance induction to bovine IgG.

In the present invention it was found that antibodies derived from milk of ruminants pass the mucosal barrier in the human intestine and can bind to an act against antigen present on the bloodstream side thereof. Without being bound by theory it is believed that antibodies derived from the milk of a ruminant binds to an Ig-receptor on human intestinal cells and is transported from the intestinal side to the bloodstream side of the intestinal mucosa. Most ruminants, pigs, and rodents have a specific neonatal receptor to transport IgG across the intestinal epithelium. This receptor is termed FcRN, the neonatal Fc receptor that is expressed shortly after birth and is downregulated by the time of weaning. Humans constitutively express FcrN on intestinal epithelia (Immunology 92:69-74) and bidirectional FcRN-dependent transport of human IgG over epithelial cells has been shown in humans (J.Clin Invest 1999 104:903-911). FcRN is also expressed on myeloid cells in humans (J.Immunol. 2001 166:3266-76).
pIgR is the polymeric Ig receptor. This receptor binds to the j-chain that is needed to form dimeric IgA and pentameric IgM. This receptor is therefore thought to transport both IgA and IgM. A recent publication has indicated that transport of antigen-complexed IgA from the intestinal lumen into the tissues can also occur (J.Gen Virol 2005 86:2747-51). CD23, the low affinity IgE receptor is also expressed on intestinal epithelium, and has been shown to play a role in the internalization of food allergens in animal models (gastroenterology 2006 131: 47-58; J.Clin Invest. 2000: 106:879-886).

The invention therefore provides a method for delivery of an antigen specific antibody of a ruminant to the mucosal side of the intestinal tract of an individual comprising providing a preparation for oral administration comprising said antigen specific antibody of a ruminant and orally administering said preparation to said individual. Ruminant antibody provided crosses the intestinal epithelium and is able to enter the intestinal mucosa and bloodstream and can interact with the antigen it is specific for.

A ruminant antibody for use in crossing the intestinal epithelium can be derived from a ruminant that has not been previously immunized for the antigen that said antibody is specific for. Alternatively, the ruminant antibody can be derived for a ruminant that has been previously immunized with the antigen that said antibody is specific for. Although ruminant antibodies are relatively resistant to degradation in the human gastro-intestinal tract it is preferred that said preparation for oral administration comprises a delivery vehicle comprising said antigen specific antibody, wherein said delivery vehicle enables targeted intestinal release of said antibody from said vehicle. Various delivery vehicles are known in the art that allow specific release of enclosed or enveloped substances in the intestinal tract. It is preferred that said vehicles allow release in the small intestine. Preferably said preparation further comprises a buffer for maintaining and/or achieving a local pH in the intestine that is favourable for uptake and/or functionality of the antibody. Preferably said buffer is set to achieve a pH between about 6-8 in the small intestine. In a preferred embodiment said antibody is obtained from the milk of a lactating ruminant. Milk of said ruminant contains, when compared for instance with serum, a large proportion ofIgG₁. IgG₁ is one of the ruminant antibody subtypes that is transported across the human intestinal mucosa. As the antibodies pass through the mucosa they can act beyond the interior of the gastro-intestinal tract. In one embodiment a ruminant antibody is specific for an allergy antigen. In this way the allergen, upon uptake by the intestine, is bound in and preferably on the bloodstream side of the intestinal mucosa thereby at least competing for binding to said antigen with human IgE specific for said antigen. In further preferred embodiment said antigen specific antibody is specific for a food allergy-associated antigen, preferably a food allergy associated antigen from soy, hazelnut, egg or apple. In a particularly preferred embodiment said allergy antigen is an allergy-associated peanut antigen. In another embodiment said allergen is an inhalation allergen or a venom allergen selected from but not limited to the group of grass pollen allergens, tree pollen allergens, house dust mite allergens, cat allergens, mould or fungus allergens, or stinging insect venoms. Said inhalation and venom allergens can enter the human body via the respiratory tract or via a sting of an insect such as a bee or a wasp.
In another preferred embodiment said in individual is suffering from an auto-immune disease or otherwise over-active immune system. In this embodiment it is preferred that said antigen specific antibody of the ruminant is specific for a proinflammatory cytokine or a stimulatory signalling factor produced by immune cells of the individual and that contribute to the maintenance and/or severity of a symptom of the auto-immune disease or otherwise over-active immune system. Thus preferably an antibody of the invention is specific for a factor excreted by immune cells. In a particularly preferred embodiment a ruminant antibody of the invention is specific for human TNF-α or human IL-23 antibody. In a preferred embodiment said individual is suffering from inflammatory bowl disease. In a further preferred embodiment said ruminant antibody of the invention is specific for an antigen of a microorganism associated with said auto-immune disease.
The human may be of any age. However, it is preferred that said human individual is at least one year old. Preferably said individual is at least two years old. Preferably said individual is at least 6 years old. In another preferred embodiment said individual is at least 16 years old. In yet another aspect of the invention said individual is between 6 and 24 months old (typically when solid food is include in the diet). In a preferred embodiment said antibody is of type IgG₁, IgA or a combination thereof. Preferably, said antibody is of the IgG₁ type or composition comprising both IgG₁ and IgA specific for said antigen. A method of the invention is preferably used to deliver said ruminant antibody to the bloodstream via the gastrointestinal tract of said individual. In a particularly preferred embodiment said bloodstream delivery is used to deliver the antibody to the lung. In this embodiment it is preferred that the antibody is specific for an antigen of a viral or a bacterial pathogen or an antigen of an allergy. In this embodiment it is preferred that said antibody is specific for a allergy-associated grass- or tree-pollen antigen, or any other inhalation allergen that causes allergic reactions in humans.
The invention thus provides the use of an antigen specific antibody of a ruminant for the preparation of a medicament for systemic treatment of a disease in a non-ruminant. Preferably said non-ruminant is a human. Preferably said medicament is for oral administration.

A particular advantage of an antibody of the invention is that it is a polyclonal antibody. This allows for more robust antigen binding. This further allows for reduced variation between individuals provided with an antibody of the invention.

In a further aspect of the invention it was found that milk from a non-human mammal that was not previously immunized for an antigen can specifically react with said antigen. This indicated to us that the milk contained antibodies that specifically bound the antigen in spite of the fact that the non-human mammal had not been immunised for said antigen. In the present invention this aspect is exploited in an industrial setting were large volumes of milk are processed. In this aspect the invention thus provides a method for obtaining an antigen specific antibody from milk characterized in that the milk is derived from a mammal that has not been immunized with said antigen prior to collecting said milk. With immunization for an antigen is meant administering a composition comprising the antigen and typically an adjuvant to a mammal with the intention of obtaining an immune response against said antigen, said adjuvant or combination thereof in said mammal. The administration involves the facilitation of intensive contact between the components of the composition and the immune cells of the mammal. Such administrations typically involve depositing the composition inside the mammal by breaking the skin layer. Other means of administration exist and can be used. Oral administration of antigen as, for instance (part of) the food of said mammal, is often associated with the induction of tolerance in the mammal for said antigen and is therefore not considered an immunization in the present invention. In a preferred embodiment said method comprises collecting milk from a lactating mammal and collecting said antibody from said milk. Preferably milk of at least two individual mammals of the same species is pooled prior to collecting said antibody. In this way, also low abundant antibodies can be collected in sufficient amounts. For this purpose it is preferred to collect antibodies from milk of at least 10 and more preferably at least 100 individual mammals preferably of the same species. In a preferred embodiment said mammal is a ruminant. Preferably said ruminant is cow, preferably a member of the genus Bos. Of the genus Bos, the commercially exploited species are preferred. Preferably said ruminant is of the species Gayal, Bos frontalis (domestic gaur), Bos mutus (Yak) or Bos taurus (Domestic Cattle). In another preferred embodiment said ruminant is a goat or a sheep, preferably a member of the subfamily Caprinae. Of this subfamily the members of the genera Ovis or Capra are preferred. Of the genus Ovis, the species Ovis aries (Domestic Sheep) is preferred. Of the genus Capra the species Capra aegagrus hircus (the domesticated goat) is preferred. In another preferred embodiment said ruminant is a camel, a donkey, a bufallo, a horse or a lama.

As milk contains several components, notably fats, oils non-antibody protein and carbohydrates it is preferred to submit said milk to a processing step prior to collecting said antibody. In a preferred embodiment said milk processing step comprises a separation step. Preferably said separation step comprises separating the milk in at least two parts and of which at least one is a protein rich part. Collection of antibody from batch processed milk is possible, however, it is preferred that said processing step is part of a (semi)-continuous process. In a particularly preferred embodiment a whey fraction is prepared from said milk and said antibody is collected from said whey. The mammal is as mentioned selected on the criterion that it has not previously been immunized for said antigen. In a preferred embodiment said mammal is selected on a further criterion for collection of said milk. This further criterion can be any criterion, for instance, time in the lactation cycle. In a preferred embodiment, said mammal is selected on the basis that it is producing normal milk, i.e. not the milk, also referred to as colostrum, immediately after giving birth. In further preferred embodiment said further criterion comprises antibody content of the milk, antibody specificity in collected milk, type of food or type of food-supplement ingestion by said mammal, vaccination for an antigen of a pathogen of said mammal or a combination of two or more of said criteria.
In a preferred embodiment said antibody is an IgG₁, IgA, IgG₂ or an IgM antibody. Preferably said antibody is collected from an antibody-enriched fraction obtained from said milk. Preferably said method further comprises a step to enhance the levels of IgG₁, IgA or a combination thereof when compared to other proteins present in the antibody enriched fraction. Preferably the amount of β-lactoglobulin is decreased by at least 50%. This can be accomplished for example by a specific hydrolysis of β-lactoglobulin, or by removal of β-lactoglobulin by size separation or by removal using an antibody specific for β-lactoglobulin. Thus preferably, a method for collecting an antibody of the invention further comprises a step to reduce the amount of β-lactoglobulin in the antibody enriched fraction. A method of the invention preferably further comprises an affinity purification step to obtain an antibody fraction that is enriched for said antigen specific antibody. Having the possibility to collect antigen specific antibody from a large collection of different mammals of the same species allows the affinity purification of significant amounts of affinity purified antigen specific antibody, in spite of a possible low prevalence of said antibody in each individual milk sample.

Further provided is an antigen specific antibody obtainable by a method for collecting an antigen specific antibody according to the invention. Preferably said antibody is a polyclonal antibody. In this embodiment said antigen specific antibody can be derived from an immunized non-human mammal or from a on-immunized non-human mammal. In a further preferred embodiment said antigen is an allergy antigen, a viral antigen, a bacterial antigen or a combination thereof.

The term antigen in the context of the present invention is an antigen of a human, a pathogen able to infect and cause disease in a human, an allergen to which a human can become allergic or a combination thereof. Immunization of a mammal in the context of the present invention thus means immunization of said mammal with an antigen of a human, a pathogen able to infect and cause disease in a human, an allergen to which a human can become allergic or a combination thereof. Vaccination of said mammal for an antigen of a pathogen of said mammal to treat or prevent a disease in said mammal is therefore not considered an immunization in the context of the present invention.

The invention further provides a composition comprising an antigen specific antibody of a non-human mammal of the invention. Preferably said antigen specific antibody comprises less than 1 % of the total amount of antibody in said composition. More preferably said antigen specific antibody comprises less than 0.1 % of the total amount of antibody in said composition. In a preferred embodiment at least 15% and more preferably at least 25% and more preferably at least 35% and more preferably at least 50% and more preferably at least 60% and more preferably at least 75% even more preferably at least 80% of the antibodies are IgG₁, IgA or a combination thereof. Preferably said composition is enriched for IgG₁ over IgA. In another preferred embodiment of the invention the composition is enriched for IgA over IgG₁.

The invention further provides a pharmaceutical composition comprising an antigen specific antibody of a non-human mammal according to the invention, or a composition according to the invention. Yet further provided is the use of an antigen specific antibody of a non-human mammal according to the invention, or a composition according to the invention, for the preparation of a medicament. In a preferred embodiment is provided the use of an antigen specific antibody of a non-human mammal according to the invention, or a composition according to the invention for the preparation of a medicament for oral administration.

In yet a further aspect the invention provides a preparation for oral administration comprising an antigen specific antibody of a non-human mammal according to the invention, or a composition according to the invention. In a preferred embodiment said preparation comprises a delivery vehicle comprising said antibody or composition. Said delivery vehicle preferably enables targeted intestinal release of said antibody from said vehicle.

In yet another aspect the invention provides a newborn or infant formula comprising a preparation according to the invention as mentioned herein above. Also provided is a newborn or infant formula comprising an antigen specific antibody of a non-human mammal according to the invention, or a composition according to the invention. Also provided is a newborn or infant formula, wherein at least 0.1, more preferably at least 1, more preferably at least 2, more preferably at least 5 and more preferably at least-10 % of the protein content of said formula is antibody protein. Preferably between about 5-10% of the protein content of said formula is antibody protein.

In yet another embodiment is provided a newborn or infant formula is indicated herein above, wherein at least 0.001, more preferably at least 0.01, more preferably at least 0.1, more preferably at least 1, more preferably at least 2, more preferably at least 5 % of the protein content of said formula is antigen specific antibody protein. Preferably between about 0.01-5% of the protein content of said formula is antigen specific antibody protein of a non-human mammal according to the invention. Thus preferably between about 0.001-5% of the protein content of said formula consists of an antigen specific antibody according to the invention, or a composition according to the invention.

The invention further provides a food supplement or a food for a non-human animal or human comprising an antibody of the invention and/or a composition of the invention. In this embodiment said antigen specific antibody can be derived from an immunized non-human mammal or from a on-immunized non-human mammal.
An antibody from an immunized or non-immunized non-human mammal is preferably specific for an allergen antigen, a human growth factor, a human antigen, a respiratory virus, respiratory bacterium, a parasite, or other relevant diseases-related antigen. In a preferred embodiment said antibody is derived from an immunized non-human mammal.
A bovine and/or non-human mammal antibody of the invention can be administered orally as a food product or neutraceutical, a drink, a capsule or an encapsulated antibody-containing preparation. A bovine and/or non-human mammal antibody from a non-immunized or from an immunized bovine and/or non-human mammal can also be administered via other routes such as via intramuscular injection, intravenous administration, or via the rectum or vagina.

An orally delivered bovine and/or non-human mammal antibody preparation can be formulated in a fluid such as a watery solution, milk, yoghurt, or as a freeze dried protein preparation, or encapsulated to protect the antibody in the stomach. Preferably, such antibody is added to an infant formula or a milk product meant for human consumption. In another preferred embodiment of the invention, said antibody is encapsulated, combined with a carrier, with a probiotic bacteria, orwith a prebiotic (e.g. galactooligosaccharides).

Further provided is a nutraceutical containing an antigen specific antibody of a non-human mammal according to the invention, or a composition according to the invention. Said nutraceutical take any form that is suitable for oral consumption. Preferably said nutraceutical is a capsule, encapsulated protein, or liquid solution. The nutraceutical is intended to prevent, treat, or at least in part alleviate symptoms of an ongoing disease. Preferably said disease is food allergy (food allergen-specific antibodies ; peanut, soy, hazelnut, egg, apple, etc), inflammatory bowel disease (TNF-α or IL-23 specific antibodies), pollen allergy (grass- and tree pollen-specific antibodies), other inhalation allergies (housedust mite, cat, fungi etc), or venom allergies.

### Examples:

### Example 1.

Effect of milk-derived antibodies from non-immunized cows on adhesion of bacteria to human epithelium. The human small intestinal epithelial cell line Caco-2 as well as the human mucin producing colon epithelial cell line HT29-MTX is cultivated in Transwell plates and allowed to differentiate into monolayers during a 15-21 day culture period. Monolayer integrity is measured every week by measuring the transepithelial electrical resistance (TEER) before use in adhesion assays. Radiolabeled bacteria (two pathogenic bacteria: enteropathogenic Escherichia coli and Salmonella typhimurium, as well as Lactobacillus.casei as a probiotic strain) are incubated with the epithelial cells at a pre-determined range of microorganisms to epithelial cells (e.g. 10:1, 100:1 and 500:1) for a set period of time and the unbound microorganisms are washed off. Adherent bacteria are quantified by d.p.m. (disintegrations per min.) counting. The experiment is performed in the presence or absence of a bovine antibody preparation at (corresponding to 464 ug/ml and 46,4 ug/ml IgG1). The adhesion of the bacteria to Caco-2 and HT29-MTX is reduced by the antibody preparation.

### Example 2.

Pathogen-specific antibody levels present in milk-derived antibody preparations.
Sandwich ELISAs to measure E.coli-specific, Salmonella-specific, and Clostridium-specific antibodies are used to quantify the amounts of bacteria-specific IgG1, IgM, and IgA in a preparation containing bovine antibodies. 96 well ELISA plates are coated with 100 µL heat killed bacteria (E.coli, Salmonella, Clostridium) in PBS. The plates are incubated overnight at 4°C in a humidified environment. The plates are washed three times with 200 ul wash buffer per well (PBS+0.05% Tween-20) and blocked for 1 hour at 37°C in a humidified environment with 200 µL block buffer per well (= wash buffer + 1% gelatin). After this incubation, the plates are washed three times with 200 ul wash buffer per well (PBS+0.05%Tween-20), and
100 µL /well of bovine antibody containing sample diluted in block buffer is added.
After a 1 hour incubation at 37°C in a humidified environment, the plates are washed three times with 200 ul wash buffer per well, and 100 µL anti-bovine IgG1-HRP (AbD Serotec, cat#AAI21P) diluted 1:50.000 in block buffer is added per well to detect bacteria-specific IgG1. For detection of IgA 100 µL anti-bovine IgA-HRP (AbD Serotec, cat# AAI20P) diluted to 1:50.000 in block buffer, and for detection of bovine IgM 100 µL anti-bovine IgM-HRP diluted to 1:50.000 in block buffer is used (AbD Serotec, cat# AAI19P).
The plates are incubated for 1 hour at 37°C in a humidified environment, washed three times with 200 ul wash buffer per well, followed by a fourth wash with PBS, and100 µL TMB substrate is added to each of the wells (100 µL TMB in 10 mL substrate buffer both from Biosource Int. Cat # 45.011.03 and 45.014.01),
The color reaction is stopped by the addition of 100 µL/well of 1M H2SO4 and the plates are read at a wavelength of 450 nm. These data demonstrate the presence of bacteria-specific bovine antibodies in a preparation of bovine antibodies from non-immunised.

### Example 3.

Allergen-specific IgG1, IgA or IgM antibodies in milk-derived antibody preparations.
Sandwich ELISAs to measure grass allergen-specific antibodies are used to quantify the amounts of grass allergen-specific IgG1, IgM, and IgA in a preparation containing bovine antibodies. 96 well ELISA plates are coated with 100 µL of grass pollen allergen extract (a mix of several temperate grasses, ALK-Abello, Horsholm, Denmark) in PBS. The plates are incubated overnight at 4°C in a humidified environment.. The plates are washed three times with 200 ul wash buffer per well (PBS+0.05°/ Tween-20) and blocked for 1 hour at 37°C in a humidified environment with 200 µL block buffer per well (= wash buffer + 1% gelatin). Following this incubation the plates are washed three times with 200 ul wash buffer per well (PBS+0.05%Tween-20), and 100 µL /well of bovine antibody containing sample diluted in block buffer is added.
After a 1 hour incubation at 37°C in a humidified environment, the plates are washed three times with 200 ul wash buffer per well, and 100 µL anti-bovine IgG1-HRP (AbD Serotec, cat# AAI21P) diluted 1:50.000 in block buffer is added per well to detect grass pollen-specific IgG1. For detection of IgA 100 µL anti-bovine IgA-HRP (AbD Serotec, cat# AAI20P) diluted to 1:50.000 in block buffer, and for detection of bovine IgM 100 µL anti-bovine IgM-HRP diluted to 1:50.000 in block buffer is used (AbD Serotec, cat# AAI19P).
The plates are incubated for 1 hour at 37°C in a humidified environment, washed three times with 200 ul wash buffer per well, followed by a fourth wash with PBS, and100 µL TMB substrate is added to each of the wells (100 µL TMB in 10 mL substrate buffer both from Biosource Int. Cat # 45.011.03 and 45.014.01)
The color reaction is stopped by adding 100 µL/well of 1M H₂SO₄, and the plates are read at a wavelength of 450 nm. The presence of grass allergen-specific bovine IgG1, IgA, and IgM is thus demonstrated in a preparation of bovine antibodies from non-immunised cows, suggesting that eating grass induces an allergen-specific antibody response in ruminants. This indicates that feeding potential food allergens to ruminants may be a way to induce (food)allergen-specific antibody responses in milk without the need to vaccinate said ruminants.

### Example 4.

Bovine milk-derived IgG1 antibodies specific for grass allergens prevent the binding of IgE-allergen complexes to CD23, the low affinity IgE receptor and prevent basophil histamine release. When allergen extracts are precomplexed to human IgE antibodies specific for theses allergens these complexes can bind to the low affinity IgE receptor CD23 (expressed on B cells, monocytes and dendritic cells) and to the high affinity IgE receptor present on basophils and mast cells. This in turn leads to highly efficient allergen presentation to Th2 cells leading to a prolonged allergic response as well as to the degranulation of basophils and mast cells. It is possible to prevent this antigen presentation as well as the release of inflammatory mediators from basophils and mastcells by introducing an allergen-specific blocking antibody of a class different from IgE, preferably an IgG antibody.
Grass pollen allergen-IgE complex binding to CD23 is investigated via the following manner. A serial dilution of grass pollen allergen extract (a mix of several temperate grasses, ALK-Abello, Horsholm, Denmark) in the absence or the presence of a bovine antibody preparation containing grass pollen allergen-specific IgG1 antibodies is prepared in a volume of 20 ul of FACS buffer (PBS, 0,1% BSA, 0,05% NaN3) in 4 ml FACS tubes. After a 30 minute incubation at 37 C, 30 uL patient serum containing grass pollen allergen-specific IgE is added and the serum and allergen are allowed to form allergen-IgE complexes for an hour at 37 °C in humidified atmosphere. After this incubation, 50.000 EBV transformed B cells that express high levels of CD23, the low affinity IgE receptor are added and the cells are incubated for one hour at 4 °C, allowing the IgE-allergen complexes to bind to CD23. After this incubation period the cells are washed with 4 mL FACS buffer and centrifuged for 5 minutes at 1,500 RPM. After washing, 50 uL of FITC-conjugated antibody to IgE (a goat anti-human polyclonal antibody; Kirkegaard & Perry Laboratories) are added to the cells in the presence of 5% normal goat serum to prevent non-specific binding of the FITC labeled anti-IgE antiserum. The tubes are incubated for half an hour at 4 °C, cells were washed with 4 mL FACS buffer and centrifuged for 5 minutes at 1,500 RPM. 200 µL FACS buffer is added per tube and the tubes are measured directly with a FACSCalibur flowcytometer. 10,000 events are recorded.
The binding of grass allergen-specific IgE to CD23 expressed on an EBV-transformed B cell line is thus demonstrated in the presence of limited amounts of grass allergen. This binding is reduced by adding a bovine milk-derived preparation containing grass pollen allergen-specific IgG1 antibodies.

Similarly it is demonstrated in a histamine release experiment from human peripheral blood basophils from grass pollen allergic patients that histamine release by the basophils is inhibited in the presence of bovine milk-derived preparation containing grass pollen allergen-specific IgG1 antibodies. In this experiment, human peripheral blood mononuclear cells (PBMC) are isolated from heparinized blood from a grass pollen-allergic patient by density centrifugation. The PBMC are collected and incubated with a serial dilution of grass pollen allergens that has been preincubated for 30 minutes at 37 C with or without a bovine milk-derived preparation containing grass pollen allergen-specific IgG1 antibodies. The PBMC are incubated for 30 minutes at 37 C, after which the supernatant is removed to measure the amount of histamine released using a commercial histamine determination kit. In this manner it is demonstrated that the release of histamine by human basophils is reduced by the presence of bovine grass pollen allergen-specific IgG1 antibodies.

### Example 5.

Transport of bovine IgG1 (and IgM, IgA) over monolayers of the human intestinal epithelium cell line Caco-2.The human small intestinal epithelial cell line Caco-2 is cultivated in Transwell plates and allowed to differentiate into monolayers during a 15-21 day culture period. Monolayer integrity is measured every week by measuring the transepithelial electrical resistance (TEER) before use in transport assays. To measure transport of bovine antibodies, a preparation containing bovine antibodies (see example 1) is added to the Caco-2 monolayers and samples are taken at several time points to measure the transport of bovine antibodies. This experiment is performed in the absence or presence of an inducer of paracellular transport (sodium caprate) or an inhibitors of transcellular transport (an 8hr pretreatment of the cells with actinomycin D). In addition, a FITC-labeled dextran is used as an additional control in all wells to visualize paracellular transport of small molecules. The antibody levels transported across the Caco-2 monolayer is measured by a sandwich ELISA developed for the measurement of total IgG1, IgM, and IgA antibodies similar to the ELISAs described in previous examples. In short, 96 well ELISA plates are coated with 100 µL sheep anti-bovine IgG diluted 1:800 in PBS (as IgG capture antibody - Bethyl Cat # A10-118A-8) in PBS, 100 µL sheep anti bovine IgA diluted in PBS per well (IgA capture antibody - Bethyl cat # A10-121A-8), or 100 µL sheep anti bovine IgM diluted in PBS per well (IgM capture antibody - Bethyl cat # A10-101A-8). The plates are incubated overnight at 4°C in a humidified environment.. Following this incubation the plates are washed three times with 200 ul wash buffer per well (PBS+0.05% Tween-20) and blocked for 1 hour at 37°C in a humidified environment with 200 µL block buffer per well (= wash buffer + 1% gelatin). The plates are washed three times with 200 ul wash buffer per well (PBS+0.05%Tween-20), and 100 µL /well of bovine antibody containing sample diluted in block buffer is added.
After a 1 hour incubation at 37°C in a humidified environment, the plates are washed three times with 200 ul wash buffer per well, and 100 µL anti-bovine IgG1-HRP (AbD Serotec, cat# AAI21P) diluted 1:50.000 in block buffer is added per well to detect the presence of bovine IgG1. For detection of IgA 100 µL anti-bovine IgA-HRP (AbD Serotec, cat# AAI20P) diluted to 1:50.000 in block buffer, and for detection of bovine IgM 100 µL anti-bovine IgM-HRP (AbD Serotec, cat# A.AI19P) diluted to 1:50.000 in block buffer is used.
The plates are incubated for 1 hour at 37°C in a humidified environment, washed three times with 200 ul wash buffer per well, followed by a fourth wash with PBS, and 100 µL TMB substrate is added to each of the wells (100 µL TMB in 10 mL substrate buffer both from Biosource Int. Cat # 45:011:03 and 45.014.01). The total amounts of bovine antibodies present in the samples are quantified against a standard curve of a standard bovine serum containing bovine IgG1, IgA, and IgM (Bethyl Cat # RS10-103).
The transport of bovine antibodies over the Caco-2 monolayer is thus demonstrated. In addition, reduction of the transport by pretreatment of the cells with actinomycin-D, and enhancement of transport by the addition of sodium caprate, an inducer of paracellular transport, indicates that bovine antibodies are actively transported over human intestinal epithelia.

### Example 6

### Uptake of bovine IgG1 (and IgA) antibodies into the human blood.

A group of six healthy human adult volunteers is selected that receive a portion of 100 ml/day of a commercially available preparation of bovine colostrum for three consecutive days. Bovine colostrums contains approximately 50 mg/ml of bovine antibodies, and a portion of 100 ml thus contains around 5 grams of bovine antibody. A blood sample is taken before the first ingestion of colostrums, and at the end of the study period. In these blood samples the amounts of bovine antibodies is measured using ELISAs that detect the presence of bovine IgG1, IgM, or IgA antibodies.
96 well ELISA plates are coated with 100 µL sheep anti-bovine IgG diluted 1:800 in PBS (as IgG capture antibody - Bethyl Cat # A10-118A-8) in PBS, 100 µL sheep anti bovine IgA diluted in PBS per well (IgA capture antibody - Bethyl cat # A10-121A-8), or 100 µL sheep anti bovine IgM diluted in PBS per well (IgM capture antibody - Bethyl cat # A10-101A-8). The plates are incubated overnight at 4°C in a humidified environment.. Following this incubation the plates are washed three times with 200 ul wash buffer per well (PBS+0.05% Tween-20) and blocked for 1 hour at 37°C in a humidified environment with 200 µL block buffer per well (= wash buffer + 1% gelatin). The plates are washed three times with 200 ul wash buffer per well (PBS+0.05°/Tween-20), and 100 µL /well of bovine antibody containing sample diluted in block buffer is added.
After a 1 hour incubation at 37°C in a humidified environment, the plates are washed three times with 200 ul wash buffer per well, and 100 µL anti-bovine IgG1-HRP (AbD Serotec, cat# AAI21P) diluted 1:50.000 in block buffer is added per well to detect the presence of bovine IgG1. For detection of IgA 100 µL anti-bovine IgA-HRP (AbD Serotec, cat# AAI20P) diluted to 1:50.000 in block buffer, and for detection of bovine IgM 100 µL anti-bovine IgM-HRP (AbD Serotec, cat# AAI19P) diluted to 1:50.000 in block buffer is used.
The plates are incubated for 1 hour at 37°C in a humidified environment, washed three times with 200 ul wash buffer per well, followed by a fourth wash with PBS, and 100 µL TMB substrate is added to each of the wells (100 µL TMB in 10 mL substrate buffer both from Biosource Int. Cat # 45.011.03 and 45.014.01). The total amounts of bovine antibodies present in the samples are quantified against a standard curve of a standard bovine serum containing bovine IgG1, IgA, and IgM (Bethyl Cat # RS10-103).
In this experiment it is shown that bovine antibodies are indeed detected in the peripheral blood of volunteers that have ingested bovine colostrum, demonstrating that bovine antibodies can enter the human bloodstream.

### Example 7.

Food allergen-specific bovine IgG antibodies can prevent allergic reactions upon ingestion of food allergens.
Cows are immunized with a peanut protein extract until sufficiently high peanut-specific antibody levels are detected in milk from the cows. Milk is collected, antibodies are isolated and purified either from whey after cheese making or directly from the milk - if possible without heating the milk.
Peanut allergic patients are selected and divided into two groups. Both groups undergo a double blind, placebo controlled food challenge to determine a threshold level for peanut sensitivity. This threshold is defined as the amount of peanut ingested that induces allergic symptoms in the patient. Following this challenge one group is placed on a diet containing an antibody preparation from peanut-immunized cows, and the control group is given a diet containing a control bovine antibody preparation (non-immunized cows). After a period of three weeks to three months on the diet all patients undergo a second double blind, placebo controlled food challenge to determine their peanut threshold.
At both timepoints blood samples are collected to determine the amount of bovine antibodies (both peanut-specific and total bovine antibody levels). These antibody levels are detected using the ELISAs described in example 6 (for total antibody levels) and the ELISAs described in example 3, with the modification that instead of grass pollen allergen extract a peanut allergen extract is coated onto the surface of the ELISA plates.
The results of this study indicate that the ingestion of peanut allergen-specific bovine antibodies increase the amount of peanut allergen a peanut allergic patient can tolerate, and suggest that oral delivery of bovine allergen-specific antibodies may be a good method to reduce allergic reactions in allergic patients. In addition, the study demonstrates that antigen-specific bovine antibodies (peanut-specific antibodies in this case) are taken up into the blood of food-allergic patients, suggesting that it is possible to systemically deliver a bovine, antigen-specific antibody to a human individual.

## Claims

1. A method for obtaining an antigen specific antibody from milk **characterized in that** the milk is derived from a non-human mammal that has not been immunized with said antigen prior to collecting said milk.

2. A method according to claim 1, comprising collecting milk from a lactating mammal and collecting said antibody from said milk.

3. A method according to claim 1 or claim 2, wherein milk of at least two individual mammals of the same species is pooled prior to collecting said antibody.

4. A method according to any one of claims 1-3, wherein said milk is submitted to a processing step prior to collecting said antibody.

5. A method according to claim 4 wherein said milk processing step comprises a separation step.

6. A method according to claim 5, wherein said separation step comprises separating the milk in at least two parts and of which at least one is a protein rich part.

7. A method according to any one of claims 4-6, wherein said processing step is part of a (semi)-continuous process.

8. A method according to any one of claims 1-7, wherein a whey fraction is prepared from said milk and said antibody is collected from said whey.

9. A method according to any one of claims 1-8, wherein said mammal is selected on a further criterion for collection of said milk.

10. A method according to claim 9, wherein said further criterion comprises antibody content of the milk, antibody specificity in collected milk, food or food-supplement ingestion by said mammal, vaccination for an antigen of a pathogen of said mammal or a combination of two or more of said criteria.

11. A method according to any one of claims 1-10, wherein said mammal is a ruminant.

12. A method according to claim 11, wherein said ruminant is a cow, a goat or a sheep.

13. A method according to any one of claims 1-12, wherein said antibody is an IgG₁, IgA, IgG₂ or an IgM antibody.

14. A method according to any one of claims 1-13, wherein said antigen is an allergy antigen, a viral antigen, a bacterial antigen or a combination thereof.

15. A method according to any one of claim 1-14, wherein an antibody enriched fraction is collected from said milk.

16. A method according to any one of claims 1-15, further comprising a step to specifically collect IgG₁, IgA or a combination thereof.

17. A method according to any one of claims 1-16, further comprising an affinity purification step to obtain an antibody fraction that is enriched for said antigen specific antibody.

18. An antigen specific antibody obtainable by a method according to any one of claims 1-17, preferably a polyclonal antigen specific antibody.

19. A composition comprising an antibody according to claim 18, wherein said antigen specific antibody comprises less than 0.1 % of the total amount of antibody in said composition.

20. A composition according to claim 16, wherein at least 15% of the antibodies are IgG₁, IgA or a combination thereof.

21. A composition according to claim 19 or claim 20 which is enriched for IgG₁.

22. A pharmaceutical composition comprising an antibody according to claim 18, or a composition according to any one of claims 19-21.

23. Use of an antibody according to claim 18 or a composition according to any one of claims 19-21, for the preparation of a medicament.

24. Use of an antibody according to claim 18 or a composition according to any one of claims 19-21, for the preparation of a medicament for oral administration.

25. A preparation for oral administration comprising an antibody according to claim 18 or a composition according to any one of claims 19-21.

26. A preparation according to claim 25, comprising a delivery vehicle comprising said antibody or composition.

27. A preparation according to claim 26, wherein said delivery vehicle enables targeted intestinal release of said antibody from said vehicle.

28. A newborn or infant formula comprising a preparation according to any one of claims 25-27.

29. A newborn or infant formula comprising an antibody according to claim 18 or a composition according to any one of claims 19-21.

30. A newborn or infant formula, wherein at least 0,1% of the protein content of said formula is antibody protein.

31. A newborn or infant formula according to claim 30, wherein at least 0,1°/ of the protein content of said formula consists of an antibody according to claim 18 or a composition according to any one of claims 19-21.

32. A method for delivering an antigen specific antibody of a ruminant to the mucosal side of the intestinal tract of an individual comprising providing a preparation for oral administration comprising said antigen specific antibody of a ruminant and orally administering said preparation to said individual.

33. A method according to claim 32, wherein said preparation for oral administration comprises a delivery vehicle comprising said antigen specific antibody, wherein said delivery vehicle enables targeted intestinal release of said antibody from said vehicle.

34. A method according to claim 32 or claim 33, wherein said antibody is obtained from the milk of a lactating ruminant.

35. A method according to any one of claims 32-34, wherein said antibody is specific for an allergy antigen.

36. A method according any one of claims 32-35, wherein said in individual is suffering from an auto-immune disease or otherwise over-active immune system.

37. A method according to any one of claims 32-36, wherein said individual is suffering from inflammatory bowl disease.

38. A method according to claim 36, wherein said antibody is specific for an antigen of a microorganism associated with said auto-immune disease.

39. A method according to claim 36, wherein said antibody is specific for a factor excreted by immune cells.

40. A method according to claim 39, wherein said antibody is an anti-TNF-α antibody or an anti-IL-23 antibody.

41. A method according to any one of claims 32-40, wherein said individual is at least one year old.

42. A method according to claim 41, wherein said individual is at least two years old.

43. A method according to any one of claims 32-42, wherein said antibody is of type IgG₁, IgA or a combination thereof.

44. A method according to any one of claims 32-43, wherein said antibody is delivered to the bloodstream via the gastrointestinal tract.

45. A method according to any one of claims 32-36, 38-44, for delivering said antibody to the lung.

46. Use of an antigen specific antibody of a ruminant for the preparation of a medicament for systemic treatment of a disease in a non-ruminant.

47. Use according to claim 46 wherein said non-ruminant is a human.

48. Use according to claim 46 or claim 47, wherein said medicament is for oral administration.
